# EUROPEAN PATENT APPLICATION

(11) **EP 2 047 885 A1**
(43) Date of publication of application: **15.04.2009**
(21) Application number: 07790892.9
(22) Date of filing: 18.07.2007
(51) Int. Cl.: A61N 1/32, A61N 1/04

(54) **LOW FREQUENCY THERAPY DEVICE AND ITS CONTROL METHOD**

(30) Priority: 28.07.2006 JP 2006206423
(71) Applicant: Omron Healthcare Co., Ltd., Kyoto 615-0084 (JP)
(72) Inventor: MIKI, Akitoshi, Kyoto-shi Kyoto 615-0084 (JP); ASAI, Rika, Kyoto-shi Kyoto 615-0084 (JP); MIZUTA, Yoshikazu, Kyoto-shi Kyoto 615-0084 (JP)
(74) Representative: Wilhelms · Kilian & Partner Patentanwälte
(86) International application number: PCT/JP2007/064137
(87) International publication number: WO 2008/013081

(57) **Abstract**

A low frequency therapy apparatus includes a pair of pads in which an area of an electrode is changeable by switching the number of electrodes to be in a conduction state. A control unit of the low frequency therapy apparatus corrects the output voltage to apply between the pads according to the area of the electrodes after change when changing the area of the electrode (number of electrodes). Specifically, the output voltage is made larger the smaller the area of the electrode. Thus, the area of the electrode can be changed without giving an uncomfortable feeling to the user.

## Description

### TECHNICAL FIELD

The present invention relates to a technique of controlling an output of a low frequency therapy apparatus.

### BACKGROUND ART

A low frequency therapy apparatus for performing treatment by flowing a low frequency current between a pair of pads attached to a body is known. Patent Document 1 proposes a low frequency therapy apparatus of a type including a single pad arranged with three or more electrodes, and in which the number and an area of a positive electrode and a negative electrode can be changed by switching a polarity of the electrode.
[Patent Document 1] Japanese Patent Publication No. 2917270 (Japanese Unexamined Patent Publication No. 9-38215)

### DISCLOSURE OF THE INVENTION

### PROBLEMS TO BE SOLVED BY THE INVENTION

A user may feel uncomfortable when an area of an electrode for outputting a low frequency current is changed. Specifically, variations in body feeling occurs such that even if a strength setting is the same, it is felt weak if the electrode area is small, and it is felt strong if the electrode area is large. When performing a therapy operation of sequentially switching the electrode area while flowing the low frequency current, a difference in body feeling becomes significant, and thus particularly becomes a problem.

In view of the above situations, it is an object of the present invention to provide a technique capable of changing the area of the electrode without giving an uncomfortable feeling to the user.

### MEANS FOR SOLVING THE PROBLEMS

The present invention adopts the following configuration to achieve the above object.

A low frequency therapy apparatus of the present invention includes a pair of pads in which an area of an electrode is changeable; and correction means for correcting an output voltage to apply between the pads according to the area of the electrode.

A change in body feeling when the electrode area is changed is assumed to be from a change in an amount of current flowing to the body due to a change in a contact resistance between the electrode and the body. In the present invention, a configuration of correcting the output voltage according to the electrode area to compensate or suppress the change in the amount of current flowing to the body is thus adopted. Variations in body feeling due to a difference in the electrode area can then be suppressed.

Specifically, the correction means increases the output voltage the smaller the area of the electrode. This is because the contact resistance becomes larger and the body feeling becomes weaker the smaller the electrode area.

The correction means corrects the output voltage using a correction coefficient prepared according to the area of the electrode; and the correction coefficient is preferably defined such that an amount of current flowing between the pads becomes constant irrespective of the area of the electrode. The uncomfortable feeling on the user then becomes small as possible. Furthermore, a complicated circuit configuration is unnecessary for realization of a correction process, which is advantageous for miniaturization and cost redaction of the low frequency therapy apparatus.

The pad preferably includes a plurality of electrodes which conduction can be switched, and changes the area by changing the number of electrodes to be in a conduction state. The electrode area can be easily changed according to such configuration.

The present invention may relate to a low frequency therapy apparatus including at least one part of the above-described means, the present invention may relate to a method of controlling the low frequency therapy apparatus including at least one part of the process, or a program for realizing such method and a recording medium recorded with the program. Each of the above-described means and processes can be combined with each other as much as possible to configure the present invention.

For instance, in the method of controlling the low frequency therapy apparatus according to the present invention, the low frequency therapy apparatus including a pair of pads in which an area of an electrode is changeable corrects an output voltage to apply between the pads according to the area of the electrode after change when changing the area of the electrode.

### EFFECT OF THE INVENTION

According to the present invention, an area of an electrode can be changed without giving an uncomfortable feeling to a user.

### BRIEF DESCRIPTION OF THE DRAWINGS

Fig. 1 is a view showing an outer appearance of a low frequency therapy apparatus;
Fig. 2 is a block diagram schematically showing a hardware configuration of the low frequency therapy apparatus.
Fig. 3 is a view showing one example of a treatment waveform using a pulse current.
Fig. 4 is a view showing one example of a treatment waveform using an alternating current.
Fig. 5 is a view showing an example of changing the number (an area) of an electrode.
Fig. 6 is a view showing an example of changing a position of the electrode.
Fig. 7 is a block diagram showing a function related to output voltage correction.
Fig. 8 is a flowchart showing a flow of process of the output voltage correction.

### DESCRIPTION OF REFERENCE SYMBOLS

- 10: Electrode switch button
- 11: Strength volume switch
- 20: Switch control function
- 21: Output voltage correction function
- 22: Correction coefficient table
- 100: Low frequency therapy apparatus
- 200: Therapy apparatus body
- 201: Operation unit
- 202: Display unit
- 203: Control unit
- 204: Current generating unit
- 205: Electrode switching unit
- 300: Pad
- 301: Electrode
- 302: Snap
- 400: Cord
- 401: Plug
- 402: Snap

### BEST MODE FOR CURRYING OUT THE INVENTION

Preferred embodiments of the present invention will be specifically described in an illustrative manner with reference to the drawings.

### <Outer appearance of low frequency therapy apparatus>

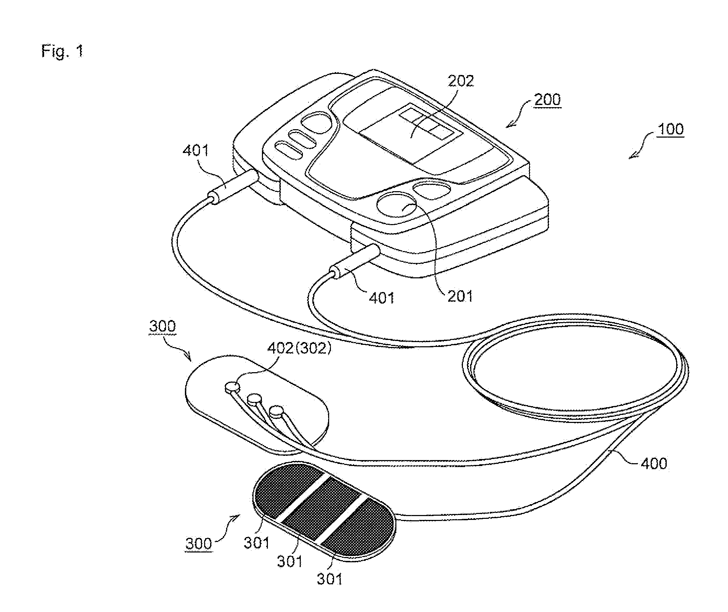

A low frequency therapy apparatus according to an embodiment of the present invention will be described with reference to Fig. 1. Fig. 1 is a view showing an outer appearance of a low frequency therapy apparatus.

A low frequency therapy apparatus 100 is schematically configured by a therapy apparatus body 200, a pair of pads 300 to be attached to a treatment site, and a cord 400 for electrically connecting the therapy apparatus body 200 and the pad 300.

The therapy apparatus body 200 is arranged with an operation unit 201 configured by a button, a volume switch and the like, and a display unit 202 configured by a liquid crystal display and the like. A user operates the operation unit 201 to switch a power ON/OFF, select a treatment mode, adjust strength and speed, switch electrodes, and perform other various settings.

The pad 300 is configured by a thin and highly flexible member. One surface (surface that contacts a body) of the pad 300 is formed with a plurality of (three) electrodes 301. A surface of each of the plurality of electrodes 301 is covered by a gel material having adherence. A film of gel material is uniformly formed over the entire surface of the pad, and the electrodes are not particularly electrically insulated by way of the film of gel material.

The other surface of the pad 300 is arranged with a snap 302 corresponding to each electrode 301. A snap 402 of the cord 400 is fastened to the snap 302 on the pad 300 side, and a plug 401 of the cord 400 is inserted to the therapy apparatus body 200 to achieve connection between the therapy apparatus body 200 and the pad 300 (electrode 301).

### <Internal configuration of low frequency therapy apparatus>

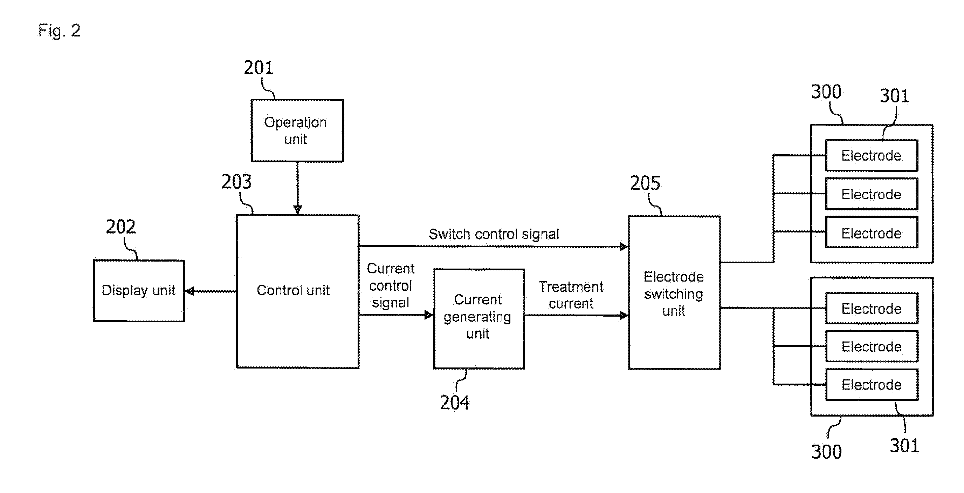

Fig. 2 is a block diagram schematically showing a hardware configuration of the low frequency therapy apparatus. As shown in Fig. 2, a control unit 203, a current generating unit 204, an electrode switching unit 205, a power supply (not shown), and the like are arranged inside the therapy apparatus body 200.

The control unit 203 is a circuit configured by a microcomputer. The signals from the operation unit 201 are input to the control unit 203. The control unit 203 has a function of controlling the display unit 202, the current generating unit 204, the electrode switching unit 205, and the like, or executing various calculation processes based on such inputs.

The current generating unit 204 is a circuit for generating a low frequency current to output from the pad 300. A pulse current may be used, an alternating current may be used, or both types of current may be selectively used for the low frequency current. A waveform and an amplitude of the low frequency current are determined by a current control signal input from the control unit 203.

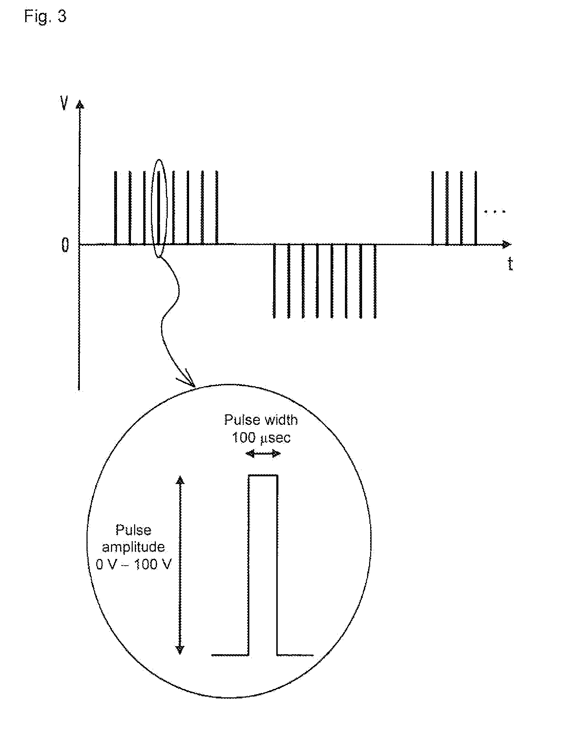

Fig. 3 shows one example of a treatment waveform using the pulse current. The pulse having an extremely short pulse width of about 100 µsec is used. A maximum value of the pulse amplitude is about 100 V. Various types of treatment mode such as massage", "pat", and "push" can be created by changing a polarity, an interval, an array, and the like of the pulse. "Strength" can be adjusted by changing the amplitude and the pulse width of the pulse, and "speed" can be adjusted by changing an appearance cycle of a pulse group.

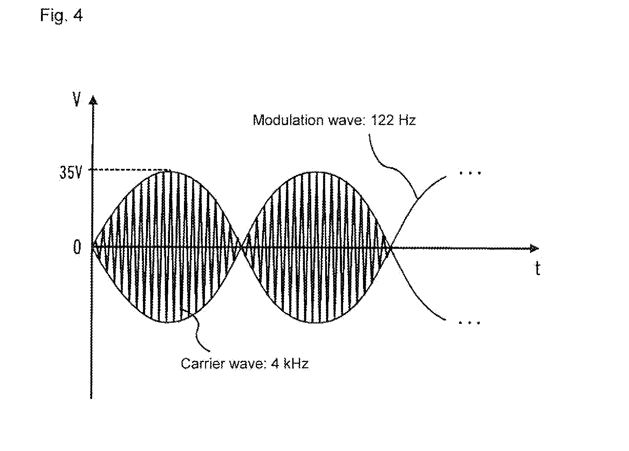

Fig. 4 shows an amplitude modulation wave in which a carrier wave frequency is about 4 kHz, a modulation wave frequency is about 122 Hz, and the maximum amplitude is about 35 V as one example of the treatment waveform using an alternating current. The "strength" is adjusted by changing the amplitude, and the "speed" is adjusted by changing the modulation wave frequency.

The electrode switching unit 205 is a circuit having a function of switching conduction/disconnection of each six electrodes 301, and distributing the low frequency current to the electrodes 301 in a conduction state. The switching of the electrode 301 is controlled by a switch control signal input from the control unit 203.

### <Operation at a time of treatment>

An operation method and a therapy operation of the low frequency therapy apparatus 100 will now be described.

When the pulse current is used, the user operates the operation unit 201 to select the treatment mode. For the treatment mode, treatment methods such as "massage", "pat", "push", and "rub", treatment sites such as "shoulder", "arm", "lower back", and the like can be specified. An "automatic" mode in which the treatment method is programmed in advance may be selected. The user can also operate a volume switch to adjust the "strength" and the "speed".

The control unit 203 generates a current control signal according to the specified treatment mode, the strength, and the speed, and sends the signal to the current generating unit 204. The current generating unit 204 performs modulation and amplification based on the current control signal, and generates the low frequency current for treatment. When the pulse current is used, the current of one system is distributed to one or a plurality of electrodes 301 of the pad on a high potential side in the electrode switching unit 205. The current flows from the electrode 301 of the pad on the high potential side to the electrode 301 of the pad on a low potential side through the body of the user. When the alternating current is used, the current of one system is distributed to each electrode 301 in the electrode switching unit 205, and is output to the body of the user through the plurality of electrodes 301. Muscles are electrically stimulated by the treatment current flowing between the pads and repeat contraction and relaxation, whereby a therapeutic effect same as massage is obtained.

### <Switching of electrode>

In the low frequency therapy apparatus 100 of the present embodiment, the plurality of electrodes 301 are arranged on each pad 300, and the conduction/disconnection of the electrode 301 can be manually or automatically switched during the output of the low frequency current. The low frequency current is distributed to one or a plurality of electrodes 301 in the conduction state, and is output to the body through the electrode 301 in the conduction state.

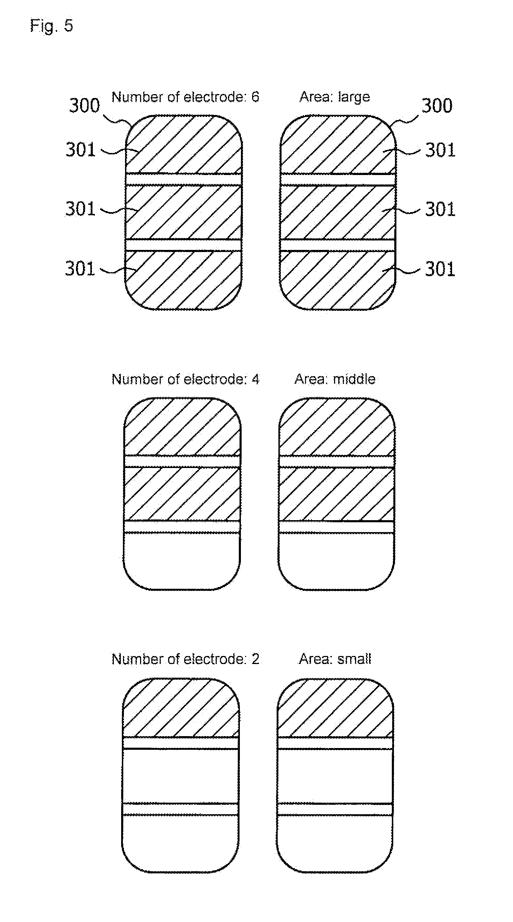

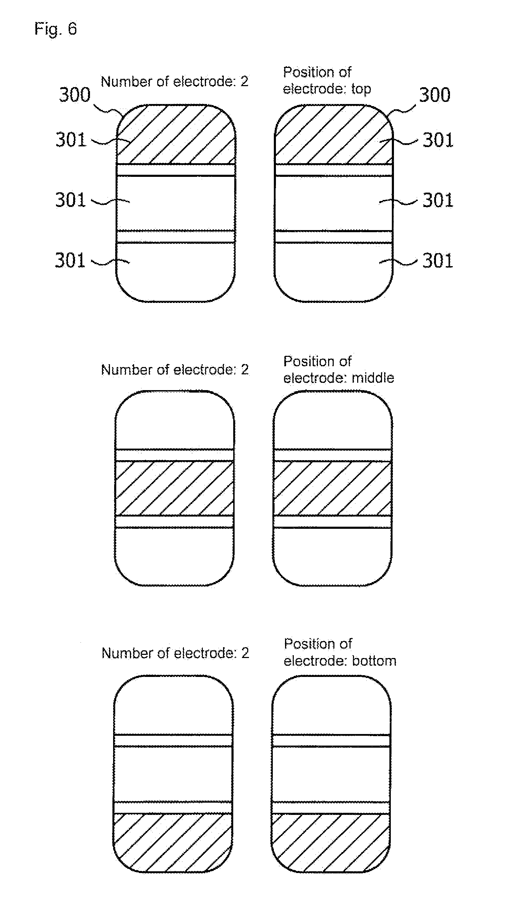

A switching example of the electrode is shown in Figs. 5 and 6. In such figures, a hatching portion represents the electrode in the conduction state. Fig. 5 is an example in which the number (an area) of the electrode is changed. If all three pairs of six electrodes 301 are conducted, the current is output from the entire pad, and thus the treatment site can be covered in a wide rang. If only one pair or two pairs of electrodes are conducted, a pinpoint treatment can be performed. Fig. 6 is an example in which the position of the electrode is changed. The treatment site can be changed by changing the position of the electrode to conduct. Thus, the range of usage becomes wider and a flexible treatment becomes possible since the plurality of electrodes are arranged on one pad.

### <Correction of output voltage>

In a conventional low frequency therapy apparatus, a strength set value and the output voltage have an one to one relationship, for example, the amplitude of the treatment current (output voltage between pads) is 10 V if the "strength" is set to "1" with the volume switch, and 50 V if the "strength" is set to "5". However, if a similar configuration is adopted in the low frequency therapy apparatus 100 of the present embodiment, a body feeling strength may change by an increase or a decrease in the electrode area even if the strength set value is the same. Specifically, the body feeling is felt weaker the smaller the area (fewer the number of electrodes in the conduction state). This is assumed to be because a contact resistance between the electrode and the body changes by the change in the electrode area, and as a result, an amount of current flowing to the body changes.

The low frequency therapy apparatus 100 of the present embodiment adopts a configuration of correcting the output voltage to apply between the pads according to the electrode area.

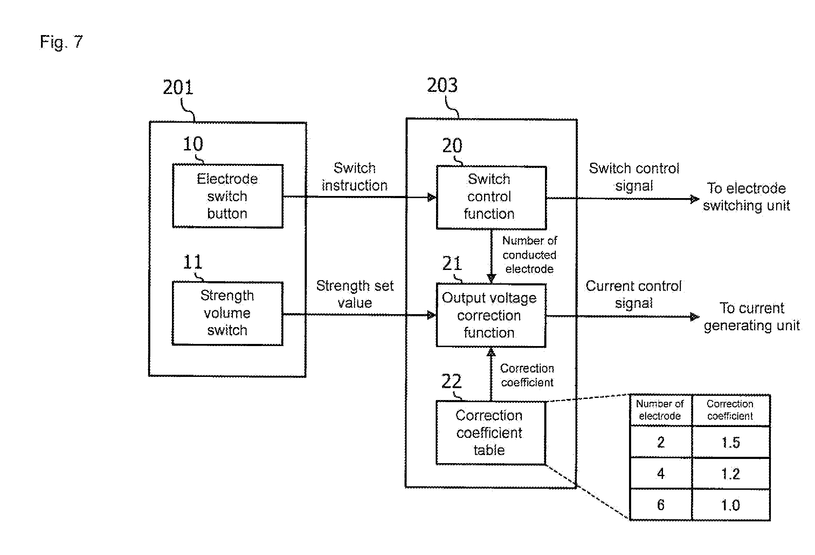

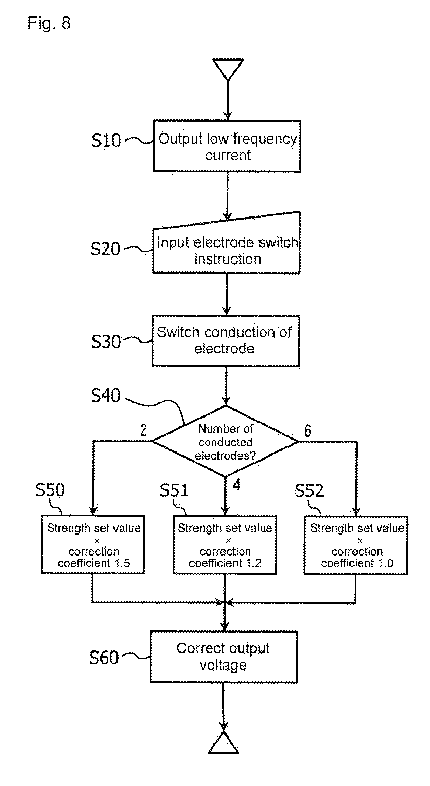

Hereinafter, the output voltage correction will be described in detail with reference to Figs. 7 and 8. Fig. 7 is a block diagram showing a function related to the output voltage correction, and Fig. 8 is a flowchart showing a flow of process of the output voltage correction.

The operation unit 201 is arranged with an electrode switch button 10 and a strength volume switch 11. The electrode switch button 10 is provided to switch the number (area) and the position of the electrode to conduct. The strength volume switch 11 is provided to adjust the strength of the treatment current.

When the user operates the electrode switch button 10 during the output of the low frequency current (step S10), a switch instruction of the electrode is input to the control unit 203 (step S20). A switch control function 20 of the control unit 203 controls the electrode switching unit 205 by outputting a switch control signal in response to the switch instruction, and switches conduction/disconnection of each electrode (step S30). The area and the position of the electrode are thereby changed.

An output voltage correction function 21 of the control unit 203 examines the number of conducted electrodes after the change, and acquires a correction coefficient corresponding to the number of conducted electrode from a correction coefficient table 22 (step S40). In the example of the present embodiment, the correction coefficients "1.5", "1.2", and "1.0" are set according to the number of electrodes "2", "4", and "6".

The output voltage correction function 21 multiplies the correction coefficient to the strength set value set in the strength volume switch 11 to calculate a strength correction value (steps S50, S51, S52). The output voltage correction function 21 sends a current control signal generated based on the strength correction value to the current generating unit 204 to correct a magnitude of the output voltage to apply between the pads (step S60). Specifically, the voltage value as in the strength set value is obtained when the number of electrodes is "6", and the voltage value of 1.2 times is obtained when "4", and 1.5 times is obtained when "2".

According to such correction, the smaller the electrode area, that is, the larger the contact resistance, the larger the output voltage becomes. Thus, the change in the output current amount caused by the change in contact resistance can be compensated or suppressed, and variation in the body feeling can be effectively suppressed.

The correction coefficient may be defined such that the amount of current flowing between the pads is constant irrespective of the electrode area. The uncomfortable feeling on the user then can be made to zero or as small as possible. It should be noted that the value of the correction coefficient mentioned in the present embodiment is merely an example. The preferred value of the correction coefficient changes depending on the shape, the number, the dividing manner, the material and the like of the electrode, and the specific numerical value may be determined through an experiment and the like.

It should be noted that the embodiment described above merely illustrates one specific example of the present invention. The scope of the present invention is not limited to the above embodiment, and various modifications may be made within the scope of the technical idea.

For instance, in the above embodiment, the output voltage is corrected after changing the electrode area, but the output voltage may be corrected first, or the change in the electrode area and the correction of the output voltage may be simultaneously executed. In the above embodiment, an example of manually switching the electrode area is shown, but the output voltage can be corrected through a similar process even when automatically switching the electrode area in the automatic mode.

The number of electrodes of the pad is not limited to three. It may be two, or more than three. The shape and the dividing manner of the electrode may be appropriately changed.

## Claims

1. A low frequency therapy apparatus comprising:
a pair of pads in which an area of an electrode is changeable; and
correction means for correcting an output voltage to apply between the pads according to the area of the electrode.

2. The low frequency therapy apparatus according to claim 1, wherein the correction means increases the output voltage the smaller the area of the electrode.

3. The low frequency therapy apparatus according to claim 1 or 2, wherein
the correction means corrects the output voltage using a correction coefficient defined according to the area of the electrode; and
the correction coefficient is defined such that an amount of current flowing between the pads becomes constant irrespective of the area of the electrode.

4. The low frequency therapy apparatus according to anyone of claims 1 to 3, wherein the pad includes a plurality of electrodes which conduction can be switched, and changes the area by changing the number of electrodes to be in a conduction state.

5. A method of controlling a low frequency therapy apparatus, wherein the low frequency therapy apparatus including a pair of pads in which an area of an electrode is changeable corrects an output voltage to apply between the pads according to the area of the electrode after change when changing the area of the electrode.
